# EUROPÄISCHE PATENTSCHRIFT

(11) **EP 1 191 016 B1**
(45) Veröffentlichungstag und Bekanntmachung des Hinweises auf die Patenterteilung: **24.03.2004**
(21) Anmeldenummer: 01810884.5
(22) Anmeldetag: 13.09.2001
(51) Int. Cl.: C07C 211/63, A01N 33/12, A61K 7/48, A61K 31/14, A61P 31/04

(54) **Neue Diquaternäre Ammoniumverbindungen**
Diquaternary ammonium compounds
Composés d'ammonium diquaternaire

(30) Priorität: 21.09.2000 EP 00810865
(43) Veröffentlichungstag der Anmeldung: 27.03.2002
(73) Patentinhaber: Ciba Specialty Chemicals Holding Inc., 4057 Basel (CH)
(72) Erfinder: Reinehr, Dieter, 79400 Kandern (DE); Ochs, Dietmar, 79650 Schopfheim (DE); Sauter, Hanspeter, 79650 Schopfheim (DE); Hoffstetter, Fernand, 68730 Ranspach le Bas (FR)

(56) Entgegenhaltungen:
- EP-A- 0 825 175
- US-A- 3 471 560
- SALVINO J M ET AL: "Structure activity relationships of non-peptide bradykinin B2 receptor antagonists" BIOORGANIC & MEDICINAL CHEMISTRY LETTERS,GB,OXFORD, Bd. 5, Nr. 4, 16. Februar 1995 (1995-02-16), Seiten 357-362, XP004135748 ISSN: 0960-894X
- KHANNA N. M. ET AL: "Studies in synthetic neuromuscular blocking agents - (IV) Synthesis of some bisalkyl onium, piperidinium, and morpholinium dihalides" J. SCI. IND. RES., Bd. 14B, 1955, Seiten 214-217, XP000981616

## Beschreibung

Die vorliegende Erfindung betrifft neue diquaternäre Ammoniumverbindungen, ein Verfahren zur Herstellung, die Verwendung als Antimikrobika gegen grampositive und gramnegative Bakterien, Hefen, Pilze, und Algen, die Verwendung als Desinfektions- und Konservierungsmittel, sowie die Verwendung zur Herstellung von Formulierungen für den technischen, kosmetischen und hygienischen Einsatz.

Die erfindungsgemässen diquaternären Ammoniumverbindungen entsprechen der Formel 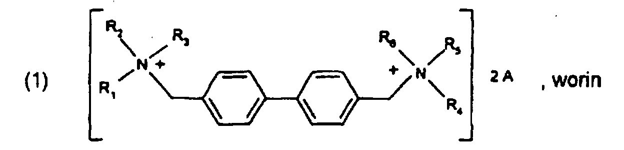 R₁ C₅-C₁₆-Alkyl, Phenyl oder Phenyl-C₁-C₁₀-Alkyl,
R₄ C₄-C₁₆-Alkyl, Phenyl oder Phenyl-C₁-C₁₀-Alkyl,
R₂, R₃, R₅ und R₆ unabhängig voneinander C₁-C₄-Alkyl, und
A ein monovalentes Anion ist,
mit der Massgabe, dass, falls R₁ und R₄ Benzyl bedeuten und R₂, R₃, R₅ und R₆ identische Bedeutungen haben und Methyl oder n-Propyl sind, A nicht für Br⁻ oder J⁻ steht.

C₄-C₁₆-Alkyl sind unverzweigte oder verzweigte Alkylreste, wie z.B. n-Butyl, sek.-Butyl, tert.-Butyl, Pentyl, Hexyl, Octyl, Decyl, Dodecyl, oder Hexadecyl.

C₅-C₁₆-Alkyl sind unverzweigte oder verzweigte Alkylreste, wie z.B. Pentyl, Hexyl, Octyl, Decyl, Dodecyl, oder Hexadecyl.

Phenyl-C₁-C₁₀-Alkyl bedeutet z.B. Benzyl, Phenethyl, Phenylpropyl, Phenylisopropyl, Phenylbutyl, Phenyl-sek.Butyl, Phenyl-tert.Butyl, oder Phenyldecyl.

C₁-C₄-Alkyl sind unverzweigte oder verzweigte Alkylreste wie z.B. Methyl, Ethyl, Propyl, Isopropyl, n-Butyl, sek.-Butyl, tert.-Butyl.

A bedeutet ein monovalentes Anion, z.B. ein Halogenidanion wie F, Cl⁻, Br⁻, I⁻, oder eine anorganische oder organische Gruppe wie z.B. Hydrogencarbonat, Nitrat, Hydrogensulfat, Chlorat, Dihydrogenphosphat, Formiat, Acetat, oder p-Toluolhydrogensulfat. Bevorzugt als Halogenidanion ist hierbei Cl⁻.

R₁, R₂, R₃, R₄, R₅ und R₆ können jeweils voneinander unabhängige Substituenten sein mit ihren angegebenen Bedeutungen. Bevorzugt sind diquaternäre Ammoniumverbindungen, in welchen R₄ und R₁, R₅ und R₂, und R₆ und R₃ identisch sind, gemäss der Formel (1'):

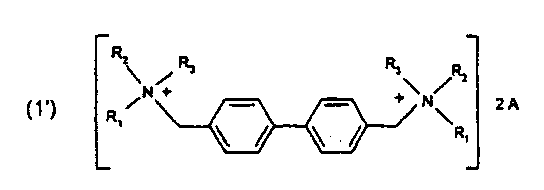

Interessant sind Verbindungen der Formel (1'), worin R₁ C₅-C₁₆-Alkyl, vorzugsweise unverzweigtes C₅-C₁₆-Alkyl, und insbesondere C₆-, C₈-, C₁₂- oder C₁₆-Alkyl bedeutet.

Weiterhin sind Verbindungen der Formel (1') interessant, worin R₂ und R₃ unabhängig voneinander unverzweigtes C₁-C₄-Alkyl, insbesondere eine CH₃-Gruppe bedeuten.

Ganz besonders interessant sind die genannten Verbindungen der Formel (1'), worin A ein Halogenidanion, insbesondere Cl⁻ bedeutet, vorzugsweise gemäss den Formeln

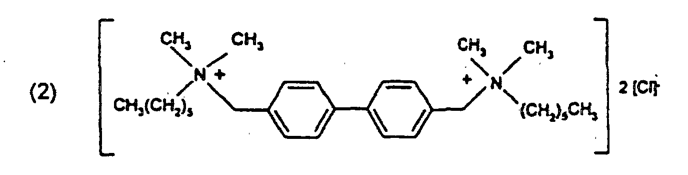

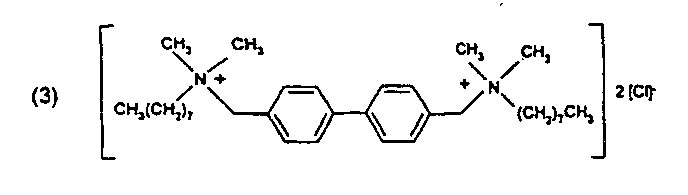

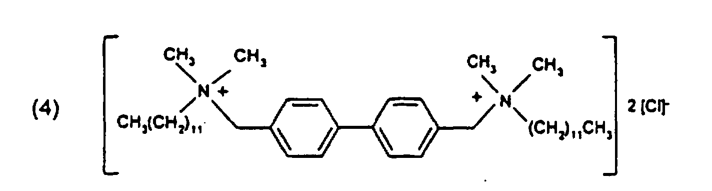

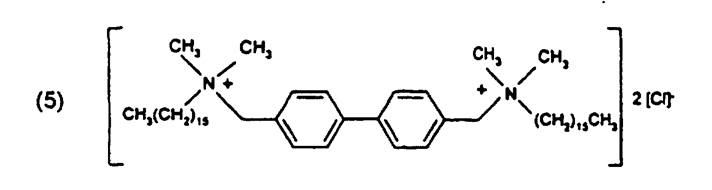

Das erfindungsgemässe Verfahren zur Herstellung der diquatemären Ammoniumverbindungen der Formeln (1) bzw. (1') besteht in einer Quatemierungsreaktion einer Biphenylverbindung der Formel 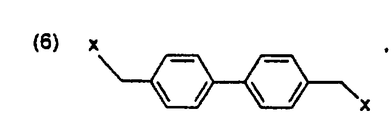 worin
X Halogen wie F, Cl, Br, I, oder eine monovalente, anorganische oder organische, anionbildende Abgangsgruppe wie z.B. -CO₃H, -NO₃, -SO₄H, -ClO₃, -PO₄H₂, -COOH, - COOCH₃, -SO₃(C₆H₄)CH₃ bedeutet,
mit einer Aminoverbindung der Formel 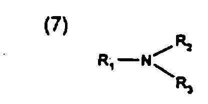 worin
R₁, R₂ und R₃ unabhängig voneinander die angegebene Bedeutung haben, sowie mit einer weiteren Aminoverbindung der Formel 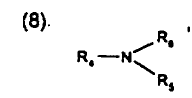 worin
R₄, R₅ und R₆ unabhängig voneinander die angegebene Bedeutung haben.

Beim allgemeinen Verfahren zur Herstellung der diquaternären Ammoniumverbindung der Formel (1) wird die Reaktion der Biphenylverbindung (6) mit zwei unterschiedlichen Aminoverbindungen (7) und (8) durchgeführt, was zu einem Gemisch von bis zu drei verschiedenen Produkten führen kann, die man gewünschtenfalls beispielsweise mittels chromatographischer Methoden auftrennen kann.

Beim Verfahren zur Herstellung der diquaternären Ammoniumverbindung der Formel (1') wird die Reaktion der Biphenylverbindung (6) beispielsweise mit einer Aminoverbindung der Formel (7) vorzugsweise in einem polaren Lösungsmittel unter Erwärmung durchgeführt, wobei als Lösungsmittel beispielsweise Wasser, Alkohole wie z.B. MeOH, EtOH oder Isopropanol, Ketone wie z.B. Aceton oder DMF, DMSO, oder eine Mischung zweier oder mehrerer dieser Lösungsmittel möglich ist. Die Aminoverbindung (7) wird vorzugsweise in stöchiometrischem Verhältnis oder in stöchiometrischem Überschuss zur Biphenylverbindung (6) eingesetzt. Besonders geeignete Aminoverbindungen der Formel (7) sind solche, in weichen R₁ unverzweigtes C₅-C₁₆-Alkyl, und R₂ und R₃ jeweils eine CH₃-Gruppe bedeuten. Beispielsweise wird N,N'-Dimethylhexylamin in Wasser vorgelegt und auf ca. 60-90°C erwärmt. Anschliessend wird die Biphenylverbindung der Formel (6), worin X bevorzugt Halogen, und insbesondere -Cl bedeutet, beispielsweise Bis-(chlormethyl)-biphenyl, in einem geeigneten Lösungsmittel, beispielsweise in Isopropanol, während ca. 40-80 Minuten zudosiert. Nach längerem Rühren (z.B. ca. 2 h) wird das Isopropanol bei ca. 80-110°C abdestilliert, wobei gleichzeitig Wasser zur Mischung zugetropft wird. Nach dem Einengen wird der Rückstand im Vakuum zur Trockene gebracht. Man erhält die diquaternäre Ammoniumverbindung, die im genannten Beispiel der Formel (2) entspricht. Nähere Einzelheiten über das erfindungsgemässe Herstellungsverfahren sind den entsprechenden Beispielen zu entnehmen.

Die beim Herstellungsverfahren eingesetzten Biphenylverbindungen der Formel (6) sind bekannt, oder können in an sich bekannter Weise hergestellt werden; beispielsweise nach der Blanc-Chloromethylierungsreaktion aus Biphenyl, Formaldehyd, und HCI in Gegenwart von ZnCI (z.B. DE-A-1793482).

Gegenstand der vorliegenden Erfindung ist ferner die Verwendung von diquatemären Ammoniumverbindungen der Formel 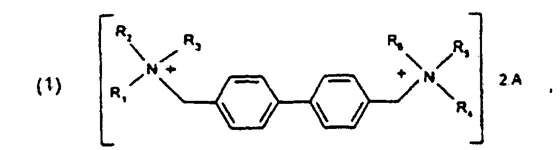 worin
R₁ und R₄ unabhängig voneinander C₄-C₁₆-Alkyl, Phenyl oder Phenyl-C₁-C₁₀-Alkyl,
R₂, R₃, R₅ und R₆ unabhängig voneinander C₁-C₄-Alkyl, und
A ein monovalentes Anion ist,
als Antimikrobika, Desinfektionsmittel oder Konservierungsmittel.

Die diquaternären Ammoniumverbindungen zeigen eine ausgeprägte antimikrobielle Wirkung, insbesondere gegen grampositive und gramnegative Bakterien, gegen Bakterien der Hautflora, gegen Hefen und Schimmelpilze, sowie gegen Algen. Sie sind daher geeignet als Antimikrobika, als Desinfektions- und Konservierungsmittel.

Sie eignen sich insbesondere zur Desinfektion, Desodorierung, sowie der allgemeinen und antimikrobiellen Behandlung der Haut, der Schleimhaut, und der Haare, besonders zur Desinfektion von Händen, Wunden, und des Rachens. Es zeigt sich ausserdem, dass die antimikrobielle Wirksamkeit auch bei geringen Konzentrationen noch gewährleistet bleibt.

Sie sind ebenfalls geeignet zur Verwendung in festen oder flüssigen Formulierungen, wie z.B. Körperpflegemittel, Shampoos, Badezusätze, Haarpflegemittel, kosmetischen und medizinischen Seifen, Lotionen, Cremes, Deodorantien, oder Reinigungslösungen, für Reinigungs- oder Spüllösungen, beispielsweise zur oralen Anwendung in Haushalten und im hygienischen Bereich, wie beispielsweise Krankenhäuser, Spitäler oder Arztpraxen, sowie für den Einsatz in Reinigungs- und Desinfektionsmitteln für Oberflächen im Haushalt, in der Industrie sowie im hygienischen Bereich.

Sie eignen sich weiter zur Ausrüstung von technischen Anlagen wie beispielsweise Kühlkreisläufe, Papierbehandlungsmaschinen, Schwimmbäder sowie zur antimikrobiellen Behandlung von Wasser zum Zwecke der Reduktion des Wachstums von Bakterien, Pilzen, Algen, und zur Schleimbekämpfung. Ausserdem eignen sie sich zur Konservierung von Materialien, wie z.B. Filtermaterialien für Luft- und Wasserfilter, textile Fasermaterialien wie z.B. Cellulosefasern, Baumwolle, Seide, Wolle, Polyamidfasem, Kunststoffe wie z.B. Verbundmaterialien, Katheter, Spritzen, sowie zur Konservierung von Papier, Holz, Leder, Farben und Lacken. Ausserdem eignen sie sich zur Konservierung von Nahrungsmitteln oder Getränken wie z.B. Bier.

Nachfolgend sind einige der genannten Anwendungen zur Herstellung von Formulierungen beispielhaft aufgeführt:

Ein Händedesinfektionsmittel hat z.B. die folgende Zusammensetzung:
0.01 bis 5 Gew.-% der Verbindung der Formel (1)
60 Gew.-% Isopropanol
0.1 Gew.-% Parfümöl, und
ad 100 % Wasser

Ein Körperpflegemittel enthält beispielsweise 0,01 bis 15, vorzugsweise 0,1 bis 10 Gew.-%, bezogen auf das Gesamtgewicht der Zusammensetzung, der diquaternären Ammoniumverbindung der Formel (1) und kosmetisch verträgliche Hilfsstoffe.

Je nachdem, in welcher Form das Körperpflegemittel vorliegt, weist es neben der diquatemären Ammoniumverbindung der Formel (1) noch weitere Bestandteile auf, wie z.B. Sequestriermittel, Farbstoffe, Parfümöle, Verdickungs- bzw. Festigungsmittel (Konsistenzregler), Emmollients, UV-Absorber, Hautschutzmittel, Antioxidantien, die mechanischen Eigenschaften verbessernde Additive wie Dicarbonsäuren und/oder Al-, Zn-, Ca-, Mg-Salze von C₁₄-C₂₂-Fettsäuren und gegebenenfalls Konservierungsmittel.

Das Körperpflegemittel kann als Wasser-in-Öl- oder Öl-in-Wasser-Emulsion, als alkoholische oder alkoholhaltige Formulierung, als vesikulare Dispersion eines ionischen oder nichtionischen amphiphilen Lipids, als Gel, fester Stift oder als Aerosol-Formulierung formuliert werden.

Als Wasser-in-Öl- oder Öl-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikonöl, einen Fettsäureester oder einen Fettalkohol. Bevorzugte Mono- oder Polyole sind Ethanol, Isopropanol, Propylenglykol, Hexylenglycol, Glycerin und Sorbitol.

Kosmetische Formulierungen können in verschiedenen Bereichen eingesetzt werden. Insbesondere kommen z.B. die folgenden Mittel in Betracht:
- Mittel zur Hautpflege, wie z.B. Hautwasch- und Reinigungsmittel in Form von stückförmigen oder flüssigen Seifen, Syndets oder Waschpasten,
- Badepräparate, wie z.B. flüssige (Schaumbäder, Milche, Duschpräparate) oder feste Badepräparate, wie z.B. Badetabletten und Badesalze;
- Hautpflegemittel, wie z.B: Hautemulsionen, Mehrfachemulsionen oder Hautöle;
- Dekorative Körperpflegemittel, wie z.B. Gesichts-Make-Ups in Form von Tages- oder Pudercremes, Gesichtspuder (lose und gepresst), Rouge oder Creme- Make-Ups, Augenpflegemittel, wie z.B. Lidschattenpräparate, Wimperntusche, Eyeliner, Augencremes oder Eye-Fix-Cremes; Lippenpflegemittel, wie z.B. Lippenstift, Lip Gloss, Lippenkonturstift, Nagelpflegemittel, wie Nagellack, Nagellackentfemer, Nagelhärter, oder Nagelhautentferner;
- Intimpflegemittel, wie z.B. Intim-Waschlotionen oder Intimsprays;
- Fusspflegemittel, wie z.B. Fussbäder, Fusspuder, Fusscremes bzw. Fussbalsame, spezielle Deomittel und Antitranspirantien oder homhautbeseitigende Mittel;
- Lichtschutzmittel, wie Sonnenmilche, -lotionen, -cremes, -öle, Sun-blockers oder Tropicals, Vorbräunungspräparate oder After-sun-Präparate;
- Hautbräunungsmittel, wie z.B. Selbstbräunungscremes;
- Depigmentierungsmittel, wie z.B. Präparate zur Hautbleichung oder Mittel zur Hautaufheilung;
- Insektenabweisende Mittel ("Repellents"), wie z.B. Insektenöle, -lotionen, -sprays, oder -stifte;
- Deodorantien, wie Deosprays, Pumpsprays, Deogele, -stifte oder -roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depilation), wie z.B. Haarentfernungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, -schäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfum, Parfum de Toilette, Parfüm), Parfümöle oder Parfümcremes;
- Mittel zur Zahn-, Zahnersatz- und Mundpflege, wie z.B. Zahncremes, Gel-Zahncremes, Zahnpulver, Mundwasserkonzentrate, Anti-Plaque-Mundspülungen, Prothesenreiniger oder Prothesenhaftmittel;
- Kosmetische Mittel zur Haarbehandlung, wie z.B. Haarwaschmittel in Form von Schampoos, Haarkonditioniermittel, Haarpflegemittel, wie z.B. Vorbehandlungsmittel, Haarwasser, Frisiercremes, Frisiergele, Pomaden, Haarspülungen, Kurpackungen, Intensivhaarkuren, Mittel zur Haarverformung, wie z.B. Wellmittel zur Herstellung von Dauerwellen (Heisswelle, Mildwelle, Kaltwelle), Haarglättungspräparate, flüssige Haarfestiger, Haarschäume, Haarsprays, Blondiermittel, wie. z.B. Wasserstoffperoxidlösungen, aufhellende Schampoos, Blondiercremes, Blondierpulver, Blondierbreie oder -öle, temporäre, semitemporäre oder permanente Haarfärbemittel, Präparate mit selbstoxidierenden Farbstoffen, oder natürliche Haarfärbemittel, wie Henna oder Kamille.

Eine antimikrobielle Seife hat z.B. folgende Zusammensetzung:
0,01 bis 5 Gew.-% der Verbindung der Formel (1)
0,3 bis 1 Gew.-% Titandioxid,
1 bis 10 Gew.-% Stearinsäure
ad 100% Seifengrundlage, wie z. B. die Natriumsalze der Talgfett- und Kokosfettsäure oder Glycerine.

Ein Schampoo hat z.B. die folgende Zusammensetzung:
0,01 bis 5 Gew.-% der Verbindung der Formel (1),
12,0 Gew.-% Natrium-Laureth-2-sulfat,
4,0 Gew.-% Cocamidopropylbetain,
3,0 Gew.-% NaCI und
ad 100% Wasser.

Ein Deodorant hat z. B. die folgende Zusammensetzung:
0,01 bis 5 Gew.-% der Verbindung der Formel (1 ),
60 Gew.-% Ethanol,
0,3 Gew.-% Parfümöl, und
ad 100 % Wasser.

Eine orale Zusammensetzung enthält beispielsweise 0,01 bis 15 Gew.-%, bezogen auf das Gesamtgewicht der Zusammensetzung, der Verbindung der Formel (1) und oral verträgliche Hilfsstoffe.

Beispiel für eine orale Zusammensetzung:
10 Gew.% Sorbitol,
10 Gew.-% Glycerin,
15 Gew.-% Ethanol,
15 Gew.-% Propylenglykol,
0,5 Gew.-% Natriumlaurylsulfat,
0,25 Gew.-% Natriummethylcocyltaurat,
0,25 Gew.-% Polyoxypropylen/Polyoxyethylen-Blockcopolymer,
0,10 Gew.-% Pfefferminzgeschmacksstoff,
0,1 bis 0,5 Gew.-% einer Verbindung der Formel (1), und
48,6 Gew.-% Wasser.

Die orale Zusammensetzung kann z.B. in Form eines Gels, einer Paste, einer Creme oder einer wässrigen Zubereitung (Mundwasser) vorliegen.

Weiterhin kann die orale Zusammensetzung Verbindungen enthalten, die Fluoridionen frei setzen, die gegen die Bildung von Karies wirksam sind, z.B. anorganische Fluoridsalze, wie z.B. Natrium-, Kalium-, Ammonium- oder Calciumfluorid oder organische Fluoridsalze, wie z.B. Aminfluoride.

Die Verbindungen können insbesondere in Haushalts- und Allzweckreinigern zur Reinigung und Desinfektion, sowie zur Desinfektion harter Oberflächen eingesetzt werden.

Ein Reinigungsmittel hat z.B. folgende Zusammensetzung:
0,01 bis 5 Gew.-% der Verbindung der Formel (1)
3,0 Gew.-% Octylalkohol 4EO
1,3 Gew.-% Fettalkohol C₈-C₁₀-Polyglucosid
3,0 Gew.% Isopropanol
ad 100 % Wasser.

Ein Allzweckreiniger hat z.B. folgende Zusammensetzung:
0,01 bis 5 Gew.-% der Verbindung der Formel (1)
2,9 Gew.-% Cocamidopropylbetain
3,0 Gew.-% Lauraminoxid
4,2 Gew.-% Natriumlaurylethersulfat
4,0 Gew.-% Natriumcitrat
3,0 Gew.-% Natriumcarbonat
3,0 Gew.-% Ethanol
ad 100 % Wasser.

Ein Allesreiniger hat z.B. folgende Zusammensetzung:
0,1 bis 5 Gew.-% der Verbindung der Formel (1 )
2,0 Gew.-% Cocamidopropylbetain
3,0 Gew.-% Lauraminoxid
6,0 Gew.-% Laurylalkohol 9 EO
4,0 Gew.-% Natriumcitrat
3,0 Gew.-% Natriumcarbonat
5,0 Gew.-% Natriumcumolsulfonat
3,0 Gew.-% Ethanol
ad 100 % Wasser.

Ein Badreiniger hat z.B. folgende Zusammensetzung:
0,01 bis 5 Gew.-% der Verbindung der Formel (1)
1,7 Gew.-% Octylalkohol 4 EO
3,5 Gew.-% Fettalkohol C₈-C₁₀-Polyglucosid
4,8 Gew.% Zitronensäure
4,0 Gew.-% Essigsäure
ad 100 % Wasser.

Ein Geschirrspülmittel hat z.B. folgende Zusammensetzung:
0,1 bis 5 Gew.-% der Verbindung der Formel (1)
15,0 Gew.-% Fettalkohol C₈-C₁₀-Polyglucosid
7,0 Gew.-% Laurylalkohol 9 EO
5,0 Gew.-% Natriumcumolsulfonat
3,0 Gew.% Zitronensäure
1,0 Gew.-% Natriumchlorid
3,5 Gew.-% Natriumsulfat
ad 100 % Wasser.

Ein weiteres Geschirrspülmittel hat z.B. folgende Zusammensetzung:
0,01 bis 5 Gew.-% der Verbindung der Formel (1)
10,0 Gew.-% Natrium C₁₄-C₁₇-Alkyl-sec.sulfonat
20,0 Gew.-% Fettalkohol C₈-C₁₀-Polyglucosid
3,0 Gew.% Laurylalkohol 9 EO
5,0 Gew.-% Natriumcumolsulfonat
3,0 Gew.-% Zitronensäure
ad 100 % Wasser.

Die nachfolgenden Beispiele erläutern die Erfindung. Darin bedeuten Teile Gewichtsteile, soweit nichts anderes angegeben ist.

Beispiele zur Synthese der diquaternären Ammoniumverbindungen der Formeln (2) bis (4):

### Beispiel 1: 4,4'-Bis-(N,N-dimethyl-N-hexyl-ammoniummethyl)-biphenyl-dichlorid (2)

25.85 g N,N'-Dimethylhexylamin (0.208 mol) wird in ca. 50 ml Wasser emulgiert und auf ca. 75°C erwärmt. Anschliessend wird eine Suspension von 25.1 g 4,4'-Bis-(chlormethyl)-biphenyl (0.1 mol) in 150 ml Isopropanol während 1 h zudosiert, wobei der pH-Wert der Mischung von anfänglich 9.4 auf 7.3 fällt. Die Mischung wird bei ca. 75-81 °C etwa 2 h gerührt, und das Isopropanol anschliessend unter Erhitzen auf ca. 81-100°C abdestilliert, wobei gleichzeitig kontinuierlich 300 ml Wasser zur Mischung zugetropft werden. Die gelbliche Lösung wird durch Zugabe von Wasser auf ein Gewicht von 510 g eingestellt (entspricht einer 10 %-igen Lösung). Die Hälfte der Lösung wird nach dem Einengen im Vakuum zur Trockene gebracht. Der Rückstand ergibt nach dem Pulverisieren 26.6 g eines weisslichen Pulvers.

Die Elementaranalyse ergibt für C₃₀H₅₀N₂Cl₂ folgende Werte:

| | C | H | N | Cl | H₂O |
|---|---|---|---|---|---|
| berechnet: | 70.42 % | 10.24 % | 5.47 % | 13.86 % | - |
| gefunden: | 67.4 % | 9.9 % | 5.2 % | 13.7 % | 3.4 % |

### Beispiel 2: 4,4'-Bis-(N,N-dimethyl-N-octyl-ammoniummethyl)-biphenyl-dichlorid (3):

Es wird verfahren wie unter Beispiel 1 beschrieben, wobei anstelle von N,N'-Dimethylhexylamin eine äquimolare Menge an N,N'-Dimethyloctylamin verwendet wird.

### Beispiel 3: 4,4'-Bis-(N,N-dimethyl-N-dodecyl-ammoniummethyl)-biphenyl-dichlorid (4):

Es wird verfahren wie unter Beispiel 1 beschrieben, wobei anstelle von N,N'-Dimethylhexylamin eine äquimolare Menge an N,N'-Dimethyldodecylamin verwendet wird.

### Beispiel 4: 4,4'-Bis-(N,N-dimethyl-N-hexadecyl-ammoniummethyl)-biphenyl-dichlorid (5):

Es wird verfahren wie unter Beispiel 1 beschrieben, wobei anstelle von N,N'-Dimethylhexylamin eine äquimolare Menge an N,N'-Dimethylhexadecylamin verwendet wird.

Bei allen vier Beispielen wird jeweils ein weissliches Pulver erhalten.

### Bestimmung der minimalen Hemmkonzentration (MHK-Wert)

Zur Bestimmung der antimikrobiellen Wirksamkeit der erfindungsgemässen Verbindungen wird wie folgt vorgegangen:

### Nährmedium:

- Casein-Sojamehl-Pepton-Bouillon zur Herstellung der Vorkulturen der Testbakterien und Hefe.
- Sabouraud-Agar zur Vorkultur von Aspergillus niger.
- Mueller Hinton Agar zur Bestimmung des MHK-Wertes von Bakterien.
- Sabouraud-Agar zur Bestimmung des MHK-Wertes von Aspergillus niger und Candida albicans.
- Deionisiertes Wasser oder EtOH als Lösungsmittel.

### Beispiele für Testkeime:

- Bakterien:: Staphylococcus hominis DSM 20328
Escherichia coli NCTC 8196
- Hefe:: Candida albicans ATCC 10231
- Schimmelpilz:: Aspergillus niger ATCC 6275

### Durchführung:

Die Testsubstanzen werden in deionisiertem Wasser oder in Ethanol vorgelöst und in einer Verdünnungsreihe in 47-50°C warmem Agar eingearbeitet.

Der Schimmelpilz wird auf Sabouraud-Agar angezüchtet und mit 0,85 % Kochsalzlösung, welche 0,01% Triton X-100 enthält, abgeschwämmt.

Bakterien und Hefe in Casein-Sojamehl-Pepton-Bouillon werden 18-24 Stunden bei 37°C inkubiert.
Alle Testkeime werden mit 0,85 %iger Kochsalzlösung auf eine Keimzahl von 10⁶-10⁷ KBE/ml eingestellt.

Jeweils 10 µl einer Keimsuspension wird auf die Agarplatten pipettiert, welche die Testsubstanz enthält. Die Testansätze werden anschliessend 2 Tage bei 37°C (Bakterien und Hefe) bzw. 3 Tage bei 28°C (Aspergillus niger) inkubiert. Zur Kontrolle des Einflusses der verwendeten Lösungsmittel (deionisiertes Wasser, Ethanol) werden zusätzlich Keimsuspensionen auf Agarplatten ohne Testsubstanzen inkubiert (Kontrollplatten).

Nach der Inkubation wird das Wachstum der Mikroorganismen auf den Testsubstanzen verglichen mit den Kontrollplatten. Als minimale Hemmkonzentration (MHK-Wert) wird diejenige Substanzkonzentration angegeben, bei der (verglichen mit den Kontrollplatten) eine deutliche Wachstumshemmung der Testkeime festzustellen ist.

Die ermittelten mikrobiologischen Daten sind in der Tabelle 1 zusammengefasst.

**Tabelle 1:**

| MHK-Werte in ppm bei verschiedenen Mikroorganismen | | | | |
|---|---|---|---|---|
| Verbindung der Formel | S. hominis | E. coli | C. albicans | A. niger |
| (2) | 50 | 5 | 50 | 2.5 |
| (3) | 5 | 1 | 10 | 2.5 |
| (4) | 10 | 250 | 25 | 100 |
| (5) | 100 | 1000 | 500 | 1000 |

### Suspensionstest zur Bestimmung der bakteriziden Aktivität nach europäischem Standard gemäss EN 1276

### Testkeime:

Staphylococcus aureus ATCC 6538.

### Testsubstanzen:

2000 ppm Lösung der Verbindung der Formel (2) und (3) in sterilem, deionisiertem Wasser.

### Kontaktzeit:

5 Minuten bei 20°C (+/- 1 °C).

### Inaktivierungsmedium nach der Kontaktzeit:

D/E Neutralisierungslösung ("Dey-Engley neutralization solution"; bestehend aus 0.1 % Natriumthioglycolat, 0.6 % Natriumthiosulfat, 0.5 % Tween 80, sowie 0.7 % Lecithin, ad 100 % sterilem, deionisiertem Wasser).

### Nährmedium:

Casein-Sojamehl-Pepton-Agar mit Lecithin, L-Histidin, und Tween 80 als Inaktivierungsmittel (Merckoplate No. 18360, Merck-Darmstadt).

### Inkubation der Testplatten:

24-48 Stunden bei 37°C.

### Zubereitung der Vorkultur:

Die Bakterienkulturen werden auf Casein-Sojamehl-Pepton Schrägagar angezüchtet und 18-24 Stunden bei 37°C inkubiert. Sie werden anschliessend mit Wasser abgeschwämmt und auf eine Keimzahl von 1.5x10⁸-5x10⁸ KBE/ml eingestellt.

### Durchführung

Alle für den Test benötigten Reagentien werden zuerst bei 20°C (+/- °C) äquilibriert. 1.0 ml 0.3 % Rinderserumalbumin (in deionisiertem Wasser) wird in ein Teströhrchen gegeben. Nach der Zugabe von 1.0 ml Keimsuspension (1.5x10⁸-5x10⁸ KBE/ml) wird die Lösung gemischt und während 2 Minuten (+/- 10 Sekunden) bei 20°C gehalten. Dann werden 8.0 ml von der Testsubstanz (2000 ppm Lösung in Wasser) zugegeben. Die Mischung wird für 5 Minuten bei 20°C (+/- °C) inkubiert. Anschliessend werden jeweils 1.0 ml der Mischung in ein Röhrchen gegeben, welches 8.0 ml an Inaktivierungsmedium (D/E Neutralisierungslösung) und 1.0 ml an deionisiertem Wasser enthält. Die Mischung wird anschliessend 5 Minuten (+/- 10 Sekunden) bei 20°C (+/- 1 °C) gehalten, und sodann 1.0 ml davon in eine Petrischale transferiert und mit 15 ml geschmolzenem Casein-Sojamehl-Pepton-Agar gemischt, welcher 3 % Tween 80, 0.3 % Lecithin, und 0.1 % L-Histidin enthält. Die Agarplatten werden 24-48 Stunden bei 37°C (+/1 °C) inkubiert. Nach der Inkubationszeit wird die Anzahl an Kolonien gezählt, und als logarithmische Reduktion (log 10) der Bakterien angegeben. Die Resultate sind in der Tabelle 2 zusammengefasst.

**Tabelle 2:**

| Suspensionstest nach EN 1276 mit Staphylococcus aureus | |
|---|---|
| Konzentration der jeweiligen Testsubstanz: 1600 ppm | |
| Kontaktzeit: jeweils 5 Minuten | |
| Logarithmische Reduktion; angegebener Wert in log 10 | |

| Verbindung der Formel | Staphylococcus aureus |
|---|---|
| (2) | 5.6 |
| (3) | 5.6 |

## Patentansprüche

1. Diquaternäre Ammoniumverbindungen der Formel 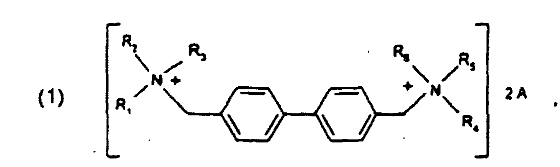 worin
R₁ C₅-C₁₆-Alkyl, Phenyl oder Phenyl-C₁-C₁₀-Alkyl,
R₄ C₄-C₁₆-Alkyl, Phenyl oder Phenyl-C₁-C₁₀-Alkyl,
R₂, R₃, R₅ und R₆ unabhängig voneinander C₁-C₄-Alkyl, und
A ein monovalentes Anion ist,
mit der Massgabe, dass, falls R₁ und R₄ Benzyl bedeuten und R₂, R₃, R₅ und R₆ identische Bedeutungen haben und Methyl oder n-Propyl sind, A nicht für Br⁻ oder J⁻ steht.

2. Diquatemäre Ammoniumverbindungen nach Anspruch 1, **dadurch gekennzeichnet, dass** R₄ und R₁, R₅ und R₂, und R₆ und R₃ identisch sind, und dass A ein Halogenidanion oder eine anorganische oder organische anionische Gruppe bedeutet.

3. Diquatemäre Ammoniumverbindungen nach Anspruch 2, **dadurch gekennzeichnet, dass** R₁ C₅-C₁₆-Alkyl bedeutet.

4. Diquaternäre Ammoniumverbindungen nach Anspruch 2 oder 3, **dadurch gekennzeichnet, dass** R₁ unverzweigtes C₅-C₁₆-Alkyl, vorzugsweise unverzweigtes C₆-, C₈-, C₁₂-, oder C₁₆-Alkyl bedeutet.

5. Diquaternäre Ammoniumverbindung nach einem der Ansprüche 2 bis 4, **dadurch gekennzeichnet, dass** R₂ und R₃ unabhängig voneinander unverzweigtes C₁-C₄-Alkyl bedeuten.

6. Diquatemäre Ammoniumverbindung nach einem der Ansprüche 2 bis 5, **dadurch gekennzeichnet, dass** A [Cl]⁻ bedeutet.

7. Diquaternäre Ammoniumverbindungen nach Anspruch 5 und 6, der Formeln

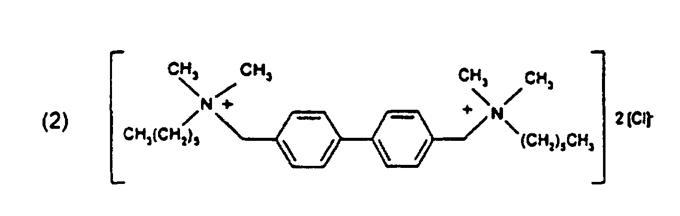

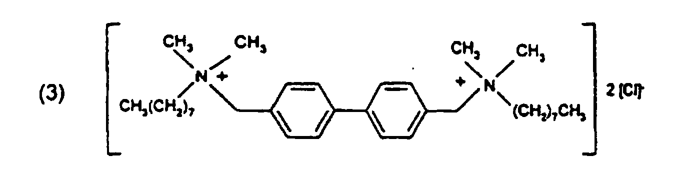

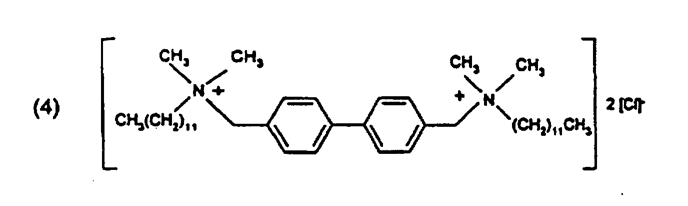

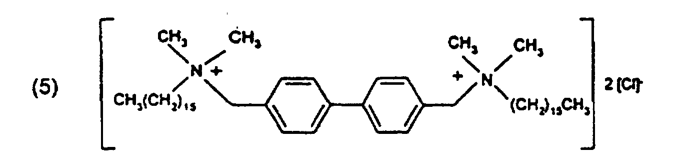

8. Verfahren zur Herstellung einer diquatemären Ammoniumverbindung nach Anspruch 1, **dadurch gekennzeichnet, dass** man eine Biphenylverbindung der Formel 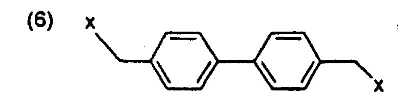 worin
X Halogen oder eine monovalente, anorganische oder organische, anionbildende Gruppe bedeutet,
mit einer Aminoverbindung der Formel 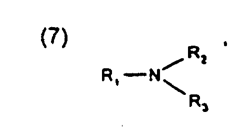 worin
R₁, R₂ und R₃ unabhängig voneinander die in Anspruch 1 angegebene Bedeutung haben, und mit einer weiteren Aminoverbindung der Formel 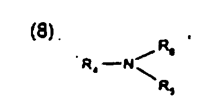 worin
R₄, R₅ und R₆ unabhängig voneinander die in Anspruch 1 angegebene Bedeutung haben, in einer Quaternierungsreaktion umsetzt.

9. Verwendung von diquaternären Ammoniumverbindungen der Formel 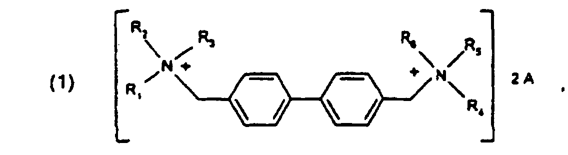 worin
R₁ und R₄ unabhängig voneinander C₄-C₁₆-Alkyl, Phenyl oder Phenyl-C₁-C₁₀-Alkyl,
R₂, R₃, R₅ und R₆ unabhängig voneinander C₁-C₄-Alkyl, und
A ein monovalentes Anion ist,
als Antimikrobika, Desinfektionsmittel oder Konservierungsmittel.

10. Verwendung nach Anspruch 9, zur antimikrobiellen Behandlung, Desodorierung oder Desinfektion der Haut, Schleimhäute oder Haare.

11. Verwendung nach Anspruch 9, zur antimikrobiellen Ausrüstung von technischen Anlagen, zur Wasserbehandlung, insbesondere von Kühlkreisläufen, Papierbehandlungsmaschinen oder Schwimmbädern, oder zur Konservierung von Materialien, insbesondere von Nahrungsmitteln oder Getränken.

12. Verwendung nach Anspruch 9, zur Herstellung einer Formulierung für den technischen, kosmetischen oder hygienischen Einsatz.

13. Verwendung nach Anspruch 9, zur Herstellung einer Flüssigformulierung zur oralen Anwendung.

14. Verwendung nach Anspruch 12, zum Einsatz in kosmetischen Produkten, zum Einsatz in Reinigungs- oder Desinfektionsmitteln für den Haushalt, für die Industrie oder für den hygienischen Bereich.

## Claims

1. A diquaternary ammonium compound of formula 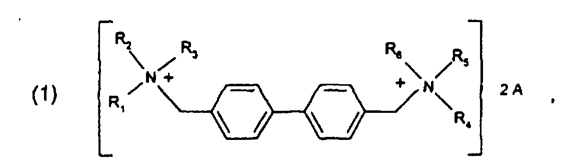 wherein
R₁ is C₅-C₁₆alkyl, phenyl or phenyl-C₁-C₁₀alkyl,
R₄ is C₄-C₁₆alkyl, phenyl or phenyl-C₁-C₁₀alkyl,
R₂, R₃, R₅ and R₆ are each independently of the others C₁-C₄alkyl, and
A is a monovalent anion,
with the proviso that when R₁ and R₄ are benzyl and R₂, R₃, R₅ and R₆ are identical and are methyl or n-propyl, A is not Br⁻ or I⁻.

2. A diquaternary ammonium compound according to claim 1, wherein R₄ and R₁, R₅ and R₂, and R₆ and R₃ are identical, and wherein A is a halide anion or an inorganic or organic anionic group.

3. A diquaternary ammonium compound according to claim 2, wherein R₁ is C₅-C₁₆alkyl.

4. A diquaternary ammonium compound according to either claim 2 or claim 3, wherein R₁ is unbranched C₅-C₁₆alkyl, preferably unbranched C₆-, C₈-, C₁₂- or C₁₆-alkyl.

5. A diquatemary ammonium compound according to any one of claims 2 to 4, wherein R₂ and R₃ are each independently of the other unbranched C₁-C₄alkyl.

6. A diquaternary ammonium compound according to any one of claims 2 to 5, wherein A is [Cl]⁻.

7. A diquatemary ammonium compound according to claim 5 and claim 6, of formula 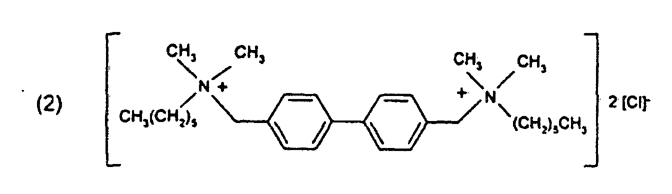 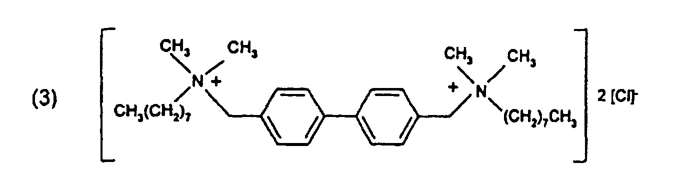 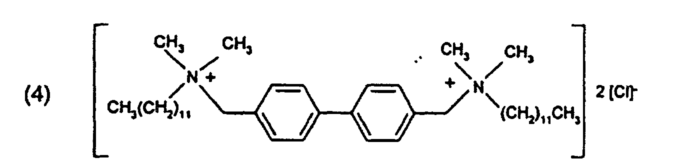 or

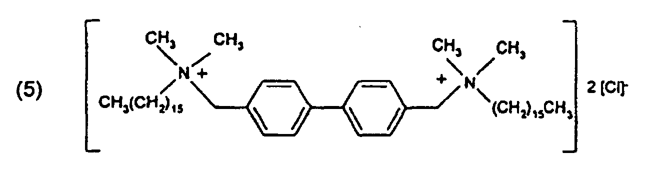

8. A process for the preparation of a diquaternary ammonium compound according to claim 1, wherein a biphenyl compound of formula 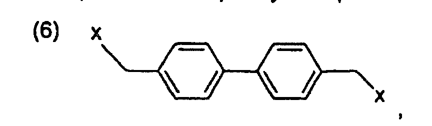 wherein X is halogen or a monovalent, inorganic or organic, anion-forming group, is reacted in a quatemisation reaction with an amino compound of formula 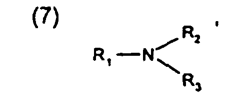 wherein R₁, R₂ and R₃ are each independently of the others as defined in claim 1, and with a further amino compound of formula 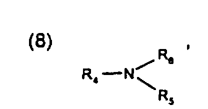 wherein R₄, R₅ and R₆ are each independently of the others as defined in claim 1.

9. Use of a diquaternary ammonium compound of formula 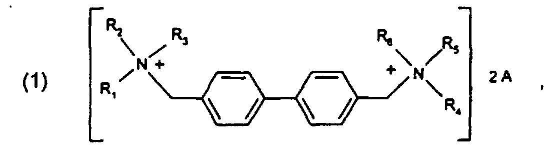 wherein R₁ and R₄ are each independently of the other C₄-C₁₆alkyl, phenyl or phenyl-C₁-C₁₀alkyl,
R₂, R₃, R₅ and R₆ are each independently of the others C₁-C₄alkyl, and
A is a monovalent anion,
as an antimicrobial agent, a disinfectant or a preservative.

10. Use according to claim 9 for the antimicrobial treatment, deodorisation or disinfection of the skin, mucosa or hair.

11. Use according to claim 9 for imparting antimicrobial properties to technical plant, for water treatment, especially cooling systems, paper treatment machines or swimming pools, or for the preservation of materials, especially foodstuffs or drinks.

12. Use according to claim 9 for the preparation of a formulation for technical use, cosmetic use or use in hygiene.

13. Use according to claim 9 for the preparation of a liquid formulation for oral use.

14. Use according to claim 12 for use in cosmetic products, or for use in cleaning or disinfecting preparations for the household sector, for industry or for the hygiene sector.

## Revendications

1. Composés d'ammonium diquaternaires de formule 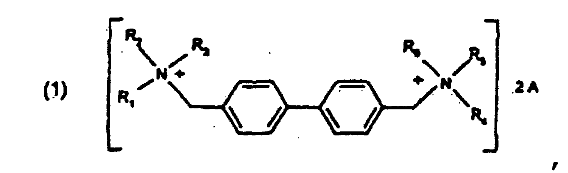 où
R₁ représente un groupe alkyle en C₅-C₁₆, phényle ou phénylalkyle en C₁-C₁₀,
R₄ représente un groupe alkyle en C₄-C₁₆, phényle ou phénylalkyle en C₁-C₁₀, et
R₂, R₃, R₅ et R₆ indépendamment les uns des autres représentent un groupe alkyle en C₁-C₄, et
A est un anion monovalent,
à condition que si R₁ et R₄ représentent un groupe benzyle et R₂, R₃, R₅ et R₆ possèdent des significations identiques et représentent un groupe méthyle ou n-propyle, A ne représente pas Br⁻ ou I⁻.

2. Composés d'ammonium diquaternaires selon la revendication 1, **caractérisés en ce que** R₄ et R₁, R₅ et R₂, et R₆ et R₃ sont identiques et que A est un anion halogénure ou un groupe anionique inorganique ou organique.

3. Composés d'ammonium diquaternaires selon la revendication 2, **caractérisés en ce que** R₁ représente un groupe alkyle en C₅-C₁₆.

4. Composés d'ammonium diquaternaires selon l'une des revendications 2 ou 3, **caractérisés en ce que** R₁ représente un groupe alkyle en C₅-C₁₆ non ramifié, de préférence un groupe alkyle en C₆, en C₈, en C₁₂ ou en C₁₆ non ramifié.

5. Composés d'ammonium diquaternaires selon l'une des revendications 2 à 4, **caractérisés en ce que** R₂ et R₃ indépendamment l'un de l'autre représentent un groupe alkyle en C₁-C₄ non ramifié.

6. Composés d'ammonium diquaternaires selon l'une des revendications 2 à 5, **caractérisés en ce que** A représente [Cl]⁻.

7. Composés d'ammonium diquaternaires selon l'une des revendications 5 et 6, de formules

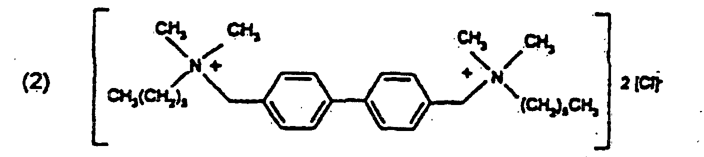

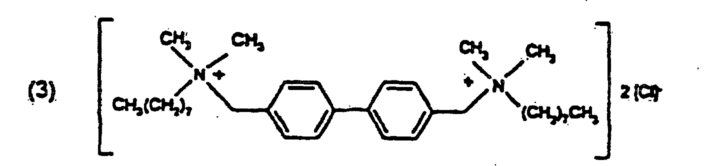

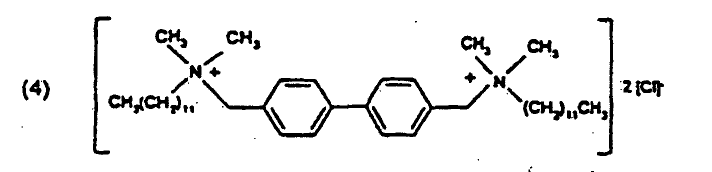

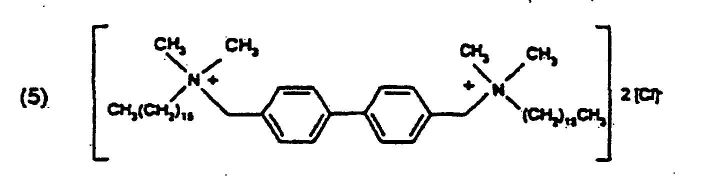

8. Procédé pour la préparation d'un composé d'ammonium diquaternaire selon la revendication 1, **caractérisé en ce qu'**on fait réagir dans une réaction de quaternisation un composé de biphényle de formule 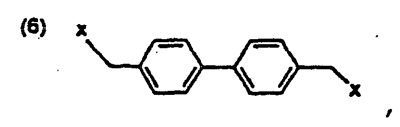 où
X représente un atome d'halogène ou un groupe anionogène monovalent, inorganique ou organique,
sur un composé aminé de formule 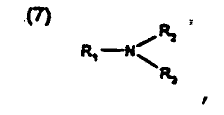 où
R₁, R₂ et R₃ indépendamment les uns des autres possèdent la signification indiquée à la revendication 1, et sur un autre composé aminé de formule 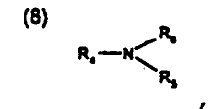 où
R₄, R₅ et R₆ indépendamment les uns des autres possèdent les significations indiquées à la revendication 1.

9. Utilisation de composés d'ammonium diquaternaires de formule 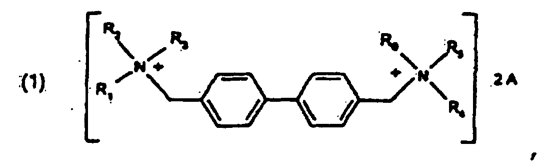 où
R₁ et R₄ indépendamment l'un de l'autre représentent un groupe alkyle en C₄-C₁₆, phényle ou phénylalkyle en C₁-C₁₀,
R₂, R₃, R₅ et R₆ indépendamment l'un de l'autre représentent un groupe alkyle en C₁-C₄ et
A est un anion monovalent,
en tant qu'agents antimicrobiens, agents désinfectants ou agents de conservation.

10. Utilisation selon la revendication 9 pour le traitement antimicrobien, pour la désodorisation ou la désinfection de la peau, des muqueuses ou des cheveux.

11. Utilisation selon la revendication 9 pour l'équipement antimicrobien d'installations techniques, de traitement d'eau, en particulier de circuits de refroidissement, de machines de traitement de papier ou de piscines, ou pour la conservation de matières, en particulier de produits alimentaires ou de boissons.

12. Utilisation selon la revendication 9 pour la préparation d'une formulation pour l'utilisation en industrie, dans des cosmétiques ou dans le domaine de l'hygiène.

13. Utilisation selon la revendication 9 pour la préparation d'une formulation liquide pour l'application orale.

14. Utilisation selon la revendication 12 pour l'utilisation dans des produits cosmétiques, pour l'utilisation dans des produits de nettoyage et des désinfectants ménagers, en industrie et dans le domaine de l'hygiène.
